# EUROPÄISCHE PATENTSCHRIFT

(11) **EP 2 528 658 B1**
(45) Veröffentlichungstag und Bekanntmachung des Hinweises auf die Patenterteilung: **25.02.2015**
(21) Anmeldenummer: 11715384.1
(22) Anmeldetag: 26.01.2011
(51) Int. Cl.: A61N 5/06

(54) **VORRICHTUNG ZUR ANWENDUNG DER ANTIBAKTERIELLEN PHOTODYNAMISCHEN THERAPIE**
DEVICE FOR USE IN ANTIBACTERIAL PHOTODYNAMIC THERAPY
DISPOSITIF À EMPLOYER POUR LA THÉRAPIE PHOTODYNAMIQUE ANTIBACTÉRIENNE

(30) Priorität: 27.01.2010 DE 102010006035
(43) Veröffentlichungstag der Anmeldung: 05.12.2012
(73) Patentinhaber: Paterok, Peter, 41061 Mönchengladbach (DE)
(72) Erfinder: Paterok, Peter, 41061 Mönchengladbach (DE)
(74) Vertreter: Thon, Sabine
(86) Internationale Anmeldenummer: PCT/DE2011/000073
(87) Internationale Veröffentlichungsnummer: WO 2011/091787

(56) Entgegenhaltungen:
- US-A1- 2008 255 498

## Beschreibung

Die vorliegende Erfindung betrifft eine Vorrichtung Anwendung der photodynamischen Therapie.

Das Prinzip der photodynamischen Therapie (PDT) basiert auf einer gezielten Photobiostimulation; so kann eine Lichtwelle von einem geeigneten Chromophor absorbiert werden. Dabei übernimmt das Chromophor die Photonenenergie des Lichtstrahles. So führt nun auch die Bestrahlung eines Photosensitizers mit einer Wellenlänge, welche dem Absorptionsmaximum entspricht dazu, dass der Farbstoff die Photonenenergie übernimmt. Hier geht der Photosensitizer in den Singulett Zustand über. Allerdings kann der angeregte Zustand in seinen Grundzustand zurückfallen, wobei er Energie in Form von Fluoreszenz abgibt. Oder er kann in einen Triplett Zustand konvertieren, in welchem er die Energie einem anderen Triplett Molekül abgeben kann. Ein solches Molekül im Grundzustand ist der Sauerstoff. Der Energietransport führt nun vom Triplett Zustand des Farbstoffes zum Sauerstoff, welcher angeregt wird und den Sauerstoff in den hoch toxischen Singulett Sauerstoff-Zustand anhebt. Ein solcher Singulett Sauerstoff ist sehr toxisch und kann massive Schädigungen am Zellkern anrichten. Allerdings ist er auch sehr kurzlebig und kann in dieser Zeit nicht weit diffundieren. Somit ist der zelluläre Schaden auf das Gebiet beschränkt, in welchem der Photosensitizer angereichert ist und das Licht einstrahlt. Die heute verwendeten Photosensitizer absorbieren Licht in den Wellenlängen zwischen 630nm und 690nm. So liegt z. B. das Absorptionsmaximum von Methylenblau bei 664 nm. Es existieren noch weitere, teilweise auch in anderen Wellenlängenbereichen wirkende Photosensitizer, so Aminolävulinsäure, Indocyaningrün, Phthalocyanin, Photolon, ein Chlorinderivat, Erythrosine, Foscan und Hypericin. Diese Aufzählung ist nicht abschließend. Die Markerkeime wurden erfahrungsgemäß am stärksten reduziert, wenn die Wellenlängen optimal angepasst sind. Auch führt die Bestrahlung alleine genauso wenig zu einer Reaktion, wie das Anfärben ebenfalls zu keiner Reaktion führt. Es kommt somit auf das Zusammenwirken von Anfärben und Bestrahlen an. Für eine optimale Dosis sind die Lichtapplikatoren für eine räumlich homogene Bestrahlung der Gewebeoberfläche und eine zuverlässige Lichtdosimetrie erforderlich.

Lichtquellen oder Lichtapplikatoren können nun nach dem jeweiligen Anwendungsbereich vielfältiger Natur sein. Es muss nur gewährleistet sein, dass Licht in der relevanten Wellenlänge auf die mit dem Photosensitizer markierten Flächen trifft.

Eine PDT Vorrichtung ist aus der US 2008/0255498 A1 bekannt.

Allgemein kann eine antibakterielle Therapie mit PDT wie folgt durchgeführt werden. Es werden ein Phorosensitizer, eine Lichiquelle, deren Wellenlänge und Leistung auf den Photosensitizer abgestimmt ist, sowie Sauerstoff in molekularer Form benötigt. Nach dem Anfärben der Mikroorganismen, so zum Beispiel Bakterien, erfolgt nach dem Wegspülen des Farbstoffüberschusses die Belichtung und Aktivierung des Photosensitizers, hierdurch wird der Singulett Sauerstoff gebildet, der die Mikroorganismen abtötet. Bekannt ist nun schon die antibakterielle photodynamische Therapie in der angewandten Zahnheilkunde, nämlich bei der Wurzelbehandlung und der Behandlung von Parodontaltaschen, also in der Parodontologie. Die Lichtquelle, die hierbei verwendet wird, ist eine sogenannte Lichtfaser, die in die Zahntaschen eingeführt werden kann. Eine solche Lichtquelle kann aber keinesfalls in allen Fällen bei der Behandlung von anderen mit Bakterien kontaminierten Körperhöblungen verwendet werden. So ist die Verwendung von Lichtfasern manchmal nicht effektiv, da zu kleine Flächen angeleuchtet werden. Des Weiteren wird durch das einfache Ausspülen zur Entfernung des Farbstoffüberschusses in manchen Körperhöhlen der bakterielle Befall in weitere versteckte Körperhöhlen transportiert. Dieser sehr nachteilige Effekt sollte unbedingt vermieden werden. So werden beim einfachen Durchspülen der Mund-, Rachen- und Nasenhöhle die Bakterien dort vollständig verteilt und somit auch in Bereiche getragen die vorher bakterienfrei waren. Weiterhin können bestimmte Körperregionen gar nicht mit Hilfe der photodynamischen Therapie behandelt werden.

Es ist daher Aufgabe der vorliegenden Erfindung eine Vorrichtung anzugeben, die eine verbesserte oder überhaupt ein photodynamische Therapie im Nasen- Mundund Rachenraums und anderen Körperstellen, die Schleimhäute oder junges Narbengewebe aufweisen, bereitzustellen, wobei die Verteilung der krankheitserregenden Keime verhindern werden soll.

Diese Aufgabe wird erfindungsgemäß durch eine Vorrichtung zur Durchführung einer antibakteriellen photodynamischen Therapie gelöst, wobei die Vorrichtung ein Lichtmittel und ein Mittel zur Abgabe des Photosensitizers aufweist, wobei das das Lichtmittel eine Lichtfaser ist, die das Licht in der gewünschten Frequenz durch seine Seitenwände abgibt und das Mittel zur Abgabe des Photosensitizers so eingerichtet ist, dass der Photosensitizer in der räumlichen Nähe der zu behandelnden Körperregion abgegeben wird und wobei die Vorrichtung eine Spülvorrichtung zum Enrfernen des Farbstoffüberschusses, also des Photosensitizers und eine Einsaugvorrichtung aufweist, die das Spülmittel und den darin gelösten Farbstoffüberschuß größtenteils wieder einsaugt und das Mittel zur Abgabe des Photosensitisers, vorzugsweise ein Schlauch, den lichtabgeberiden Teil des Lichtmittels umhüllt, wobei das umhüllende Material zumindest teilweise das von dem Lichtmittel abgegebene Licht durchlässt.

Diese Vorrichtung ermöglicht eine nahezu optimale Durchführung der photodynamischen Therapie, da hier der Photosensitizer in der räumlichen Nähe der zu behandelnden Körperregionen abgegeben wird. Es werden somit schnell die Zellen, also die Bakterien- und Mikroorganismuszellen, angefärbt, welche dann durch die Bestrahlung vernichtet werden sollen. Der Photosensitizer wird somit nicht "verschwendet" oder an Orte im Körper gebracht, an welchen er nicht seine Wirkung entfalten soll. In der erfindungsgemäßen Vorrichtung besteht das Lichtmittel zumindest teilweise aus einer Lichtfaser, welche das Licht in der gewünschten Frequenz an die zu behandelnde Körperregion abgibt. Solche Lichtfasern sind Licht emittierende Vorrichtungen, welche eine Lichtquelle urnfassen, die von dem damit zu beleuchtenden Bereich beabstandet angeordnet sind. Das heißt, die Lichtquelle erzeugt das Licht, welches dann an einer anderen, meist weiter entfernteren Stelle leuchtet. In der Regel umfassen die Lichtfasern einen Kern im zentralen Bereich und eine Umhüllung mit einem definierten Brechungsindex, der beispielsweise niedriger als der des Kerns sein kann, auf der Peripherie des Kerns. Es sind Lichtfasern benannt, die Licht bis an das Ende der Faser transportieren. Lichtfasern der erfindungsgemässen Vorrichtung geben das Licht durch die Seitenwände ab und beleuchten so die gesamte Strecke, an welcher die Lichtfaser entlang läuft. So sind Lichtfasern in der JP-A-6-118244 offenbart, welche das Licht aus dem gesamten oder auch nur aus einem bezeichneten, also eingeschränkten Seitenbereich austreten lassen. Diese Lichtfasern sind mit einem Kern und einer Umhüllung ausgestattet, welche aus transparenten Materialien hergestellt sind, wobei der Kern ein Polymer das hauptsächlich aus einem Polymethylacryltat besteht. Die Umhüllung ist aus zwei verschiedenen Bereichen hergestellt, wovon einer aus einem Copolymer auf der Basis von Vinylidenflourid besteht, das 50 bis 90 Mol% Vinylidenfluorid und 10 bis 50 Mol% Tetrafluorethylen umfasst. Der andere Bereich umfasst zumindest eines der anderen Polymere mit einem Brechungsindex, der höher ist als der des Copolymers auf der Basis von Vinylidenfluorid, vorzugsweise ein Polymer, das gleich oder ähnlich ist wie das kernbildende Polymer. Es existieren aber viele weitere Stoffe, welche die gewünschten Eigenschaften der jeweiligen Lichtfasern aufweisen. So wird in der JP-A-10-142428 ein Lichtilluminationsstab, in der JP-B-4-70604 Lichtfasern aus biegsamen Elementen offenbart.

In einer weiteren ganz besonders bevorzugten Ausführungsform der Erfindung stellt ein Schlauch oder ein schlauchförmiges Gebilde das Mittel zur Abgabe des Photosensitizers dar, welcher den Photosensitizer von einer Quelle über den Schlauch hin zu den zu behandelnden Körperregionen leitet, wobei der Schlauch mindestens einen Auslass zur Abgabe des Photosensitizers, aber in seiner Maximalanzahl unbegrenzt viele Ausläße zur Abgabe des Photosensitizers aufweisen kann.

Dieser Schlauch oder dieses schlauchförmige Gebilde beinhaltet den Photosensitizer und führt diesen an die bestimmten Körperregionen. Der Schlauch oder das schlauchförmige Gebilde kann mehrere Ausläße für den Photosensitizer aufweisen, durch welche dieser dann in das umliegende Gewebe abgegeben werden kann. Diese Ausführungsform der Erfindung gewährleistet eine genaue Abgabe und Dosierung des Photosensitizers an den bestimmten Körperregionen.

In der Ausführungsform der vorliegenden Erfindung umhüllt das Mittel zur Abgabe des Photosensitizers, vorzugsweise ein Schlauch, den lichtabgebenden Teil des Lichtmittels, wobei das umhüllende Material zumindest teilweise das von dem Lichtmittel abgegebene Licht durchlässt. Es liegt also eine weitere Ummantelung der Lichtmittels vor. Handelt es sich bei dem Lichtmittel um eine Lichtfaser, so liegen nun um den Kern und der Umhüllung des Kerns aus dem oben genannten transparenten Material eine weitere Umhüllung vor, welche den Photosensitizer in sich führt. Diese Umhüllung darf nun nicht die optischen Eigenschaften der Lichtfaser so verändern, dass das abgegebene Licht in einem anderen Wellenlängenbereich austritt und damit die Wirkungsweise der photodynamischen Therapie in Frage stellt.

In einer weiteren Ausführungsform der vorliegenden Erfindung wird das Lichtmittel, vorzugsweise eine Lichtfaser mit dem Photosensitizer beschichtet, welcher sich dann langsam von diesem ablöst und an die Mikroorganismen und Bakterien heftet.

In einer ganz anderen Ausführungsform der Erfindung ist die vorliegende Erfindung dadurch gekennzeichnet, dass das umhüllende Material aus einem Gewebe besteht, welches den Photosensitizer aufgenommen hat und diesen dann wieder an die zu behandelnden Körperregionen abgibt. So kann man sich ein Gewebenetz vorstellen, welches eine Lichtfaser umhüllt. Dieses Gewebe wird zur Vorbereitung in dem Photosensitizer gebadet, wobei es sich mit diesem voll saugt. Bei der Anwendung im Rahmen einer photodynamischen Therapie wird der Photosensitizer langsam an das umliegende Gewebe abgegeben, wo er sich verteilt. Die Richtung der Verteilung innerhalb des umliegenden Gewebes wird durch die dort vorhandene mögliche Strömung und durch Diffusionskräfte bewirkt.

In der Ausführungsform der Erfindung weist die erfindungsgemäße Vorrichtung (Applicator) zusätzlich noch eine Spülvorrichtung zum Entfernen des Farbstoffüberschusses auf. Eine solche Vorrichtung (Applicator), also eine Ausführungsform zur Durchführung der antibakteriellen photodynamischen Therapie, die extra an die zu behandelnden Körperstellen angepasst ist, unterstützt dank der Kombination der Spülvorrichtung und der Lichtquelle die Wirksamkeit der Behandlung. Die räumliche Nähe von Spülvorrichtung und Lichtquelle gewährleistet eine zielgenaue Behandlung an dem gewünschten Ort. Auch kann die zeitliche Abfolge von Spülung und Belichtung exakt aufeinander abgestimmt werden. Hierbei muss zusätzlich noch beachtet werden, dass bei einer Spülung mit Wasser, dieses ebenfalls als Lichtleiter dienen kann. Dieser sogenannte Tyndall-Effekt sollte bei schwierig erreichbaren Stellen genutzt werden.

In der Ausführungsform weist die erfindungsgemäße Vorrichtung (Applicator) zur Durchführung einer antibakteriellen photodynamischen Therapie die Spülvorrichtung und eine Einsaugvorrichtung auf, die das Spülmittel größtenteils wieder einsaugt. Das Entfernen des Farbstoffüberschusses muss sorgfältig durchgeführt werden, da dieser die Belichtung und Aktivierung sogar behindern kann, wenn er in zu dicken Schichten auf dem Gewebe liegt. Der Farbstoff sollte bei einer optimalen Behandlung vom Gewebe in die Zellwand aufgenommen werden. Jeglicher Farbstoff, der nicht von der Zellwand aufgenommen wurde, sollte entfernt werden. Dies geschieht wie schon mehrfach erwähnt durch einfaches Ausspülen. Wasser eignet sich hierbei als Spülmittel. Allerdings kann zu heftiges Ausspülen die Bakterien auch in Körperöffnungen spülen, in welchen sie vorher noch nicht präsent waren und dadurch sogar zu einer Verbreitung des Bakterienbefalls führen. Wird nun direkt nach dem Spülen das Spülmittel, z. B. Wasser wieder eingesogen, so kann sich dieses nicht verbreiten. Sind Spül- und Einsaugvorrichtung in einer direkten Nähe, so wird der Farbstoffüberschuss auch dort eingesaugt.

In einer ganz besonderen Ausführungsform der erfindungsgemäßen Vorrichtung (Applicator) weist dieser einen Ultraschall-Vernebler auf, der das Spülmittel in einer sehr fein verteilten Form auf die gewünschten Stellen aufbringt. Hierdurch kann die Menge an eingebrachtem Spülmittel besser dosiert und sogar bei gleicher Wirkung reduziert werden. Ultraschall-Vernebler sind in der gewünschten Größe im Handel erhältlich und können leicht in die Vorrichtung (den Applicator) eingebaut werden. Die fein verteilten Spülmittelteilchen sorgen sogar noch dafür, dass bei der Belichtung das Licht gestreut und dadurch wirkungsvoller die gefärbten Zellwände bestrahlt.

In einer weiteren Ausführungsform ist die Vorrichtung (der Applicator) y-förmig ausgestaltet, so dass er sich für den Einsatz im Nasenraum eignet. Jeder der beiden Enden des y-förmigen Applicators (der Vorrichtung) weist eine eigene Spülvorrichtung und eine eigene Lichtquelle auf. Der Applicator (die Vorrichtung) wird nun so in die Nase eingeführt, dass jeweils ein Ende in jedem Nasenloch ist. Dies stellt sicher, dass die gesamte Nasenschleimhaut gleichzeitig behandelt wird.

In einer ganz besonderen Ausführungsform der vorliegenden Erfindung ist diese dadurch gekennzeichnet, dass die Spül- und Einsaugvorrichtung des einen Applicatorendes (des Endes der Vorrichtung) dann spült, wenn die Spül- und Einsaugvorrichtung des anderen Applicatorendes das Spülmittel einsaugt. Dies sorgt für eine Flussrichtung des Spülmittels von dem einen Applicatorende zum dem anderen Applicatorende und vermeidet die Verteilung des mit Bakterien kontaminierten Spülmittels in dem gesamten Nasenraum, den Nasennebenhöhlen und den anderen dort vorhandenen Öffnungen. Durch einen Wechsel der Flussrichtung wird der Spülvorgang sogar noch effektiver.

In einer weiteren Ausführungsform der vorliegenden Erfindung ist die Spülvorrichtung als Inhalationsvorrichtung ausgestaltet. Hierdurch wird das Spülmittel durch Inhalation eingesogen und spült dann den Farbstoffüberschuss weg.

In einer anderen Ausführungsform der vorliegenden Erfindung weist die Vorrichtung (der Applicator) eine an die Zahnleiste angepasste Form auf und dient somit zu optimalen Anwendung im Rachenbereich. Auch in dieser Ausführungsform können Spül- und Einsaugvorrichtung an den jeweiligen Applicatorenden (Vorrichtungsenden) angebracht sein und bei wechselseitigem Einsatz eine Flussrichtung des Spülmittels vorgeben. Diese Ausführungsform kann wie eine Aufbeißschiene ausgestaltet sein und zudem noch einen spatelförmigen Fortsatz aufweisen, der dazu dient die Zunge herunter zu drücken, damit die Behandlung ungehindert durchgeführt werden kann.

Eine weitere Ausführungsform des erfindungsgemäßen Applicators (Vorrichtung) ist zylinderförmig ausgestaltet und kann so zur effektiven Behandlung der Schleimhäute im rektalen, analen und vaginalen Bereich dienen.

Die folgenden nicht einschränkend zu verstehenden Ausführungsbeispiele sollen den Einsatz der Vorrichtung verdeutlichen.

An allen Körperregionen und Körperstellen, an welchen sich bakterielle Entzündungen bilden können, also bevorzugt solche Regionen, an welchen sich Schleimhäute befinden, kann die erfindungsgemäße Vorrichtung eingesetzt werden. Nähte, welche zertrennte Gewebeteile wieder zusammenfügen sollen, können mit fadenförmigen Vorrichtungen durchgeführt werden. Diese so beschaffenen Nähte sondern den Photosensitizer ab, welcher sich dann an die auftretenden Bakterien anlagert. Ein Ende dieser Nähte wird nun mit einer geeigneten Lichtquelle verbunden. Das transportierte Licht tritt entlang der Naht aus und die angefärbten Bakterien und Mikroorganismen werden erfolgreich bekämpft.

Katheter, zumindest alle in den Körper einführbaren Katheter bsp Urinkatheter sowie Endoskope, Kanülen, Stents, sonstige Implantate, also Fremdkörper, die in den Körper eingeführt werden und dort Keime anlagern können oder Entzündungen hervorrufen können, kann man nun so fertigen, dass das Produkt der erfindungsgemäßen Vorrichtung entspricht. Diese so gefertigten Vorrichtungen werden dann mit einer geeigneten Lichtquelle verbunden und potentielle Entzündungen können erfolgreich bekämpft werden.

Die erfindungsgemäße Vorrichtung kann somit wie oben beschrieben eine Vielzahl an Formen annehmen, die alle dem zu behandelnden Körper nachempfunden sein können. Einige Ausführungsformen sind Vollkern-, der zylindrische Hohlkörper-, der schlauchförmige -, und der plattenförmige folienartige Applicator sind hier beispielhaft zusammen fassend zu nennen.

Die Verteilung des Photosensitizers kann per Spülung bzw. Saugung, per Diffusion bei dem imprägnierten Applicator und per Inhalation bzw. Saugung vorgenommen werden.

Die erfindungsgemäße Vorrichtung bzw. die Applicatoren finden ihren Einsatz bei Haut-/Schleimhautoberflächen (Brandwunden, Wunden, zugängliche Schleimhäute sind die oberen Atemwege, z. B. Rachen, Darm, Vagina, ableitende Harnwege, andere Endoscopisch erreichbare Körperregionen oder aber Körperregionen, die anders z.B. durch Mikroroboter applizierbar sind. Weiterhin sind noch die oberen - und weiteren Bereiche des Ernährungstraktes wie Speiseröhre, Magen, Zwölffingerdarm etc. als Einsatzgebiet zu nennen. Äußerlich ist die erfindungsgemäße Vorrichtung auf der Haut zu verwenden. Die inneren Hohlorgane wie der Magen, Darm .. etc sind ebenfalls zu den Einsatzgebieten zu zählen. Auch die Gefäße sind durch entsprechend angepasste Kanülen o. a. Applicatoren ein Einsatzgebiet für die erfindungsgemäße Vorrichtung.

Im folgenden werden nicht einschränkend zu verstehende Ausführungsbeispiele der vorliegenden Erfindung anhand der Zeichnung besprochen. In dieser zeigen:

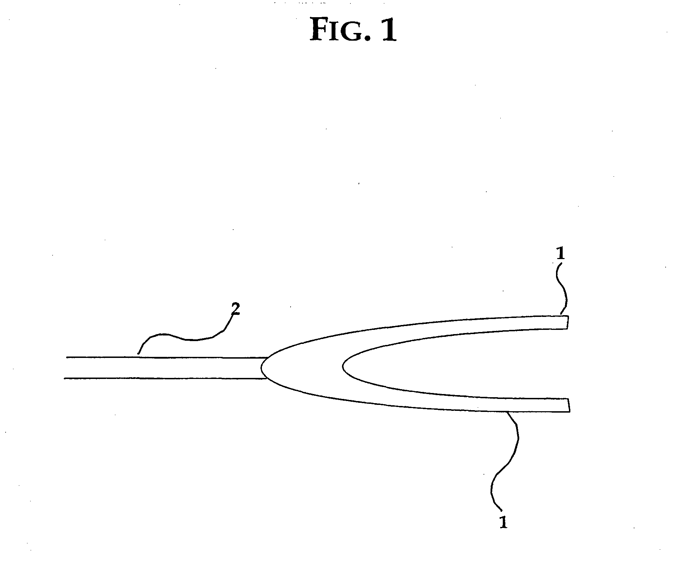

Fig. 1 schematisch einen y-förmigen Applicator

Fig. 1 zeigt schematisch einen y-förmigen Applicator. Dieser eignet sich für die Anwendung der photodynamischen Therapie in Nasenraum. Hierzu wird das eine Ende des Y-Fortsatzes 1 in das rechte Nasenloch das andere Ende des Y-Fortsatzes in das linke Nasenloch geschoben. In der Figur ist auch die Zuleitung 2 für die Energie- und Spülmittelversorgung schematisch dargestellt. Nun kann die Nasenschleimhaut, nachdem diese mit dem Photosensitizer gefärbt wurde, mit dem Spülmittel ausgespült werden, um den Überschuss an Farbe auszuspülen. In beiden Enden des Y-Fortsatzes des Applicators befindet sich eine Spülvorrichtung, eine Saugvorrichtung und die Lichtquelle. Damit das Spülmittel nicht die Bakterien in andere Regionen des Körpers trägt, wird nun mit dem einen Ende des Applicators gespült mit dem anderen Ende jedoch das Spülmittel direkt wieder eingesaugt. Dadurch kann sich das mit den Bakterien kontaminierte Spülmittel nicht weiter räumlich verteilen und neue infizierte Herde ausbilden. Der Spülmittelfluss wird somit ausgerichtet und gelenkt.

## Patentansprüche

1. Vorrichtung zur Durchführung einer antibakteriellen photodynamischen Therapie, wobei die Vorrichtung ein Lichtmittel und ein Mittel zur Abgabe des Photosensitizers aufweist, und wobei
• das Lichtmittel eine Lichtfaser ist, die das Licht in der gewünschten Frequenz durch seine Seitenwände abgibt und
• das Mittel zur Abgabe des Photosensitizers so eingerichter ist, dass der Photosensitizer in der räumlichen Nähe der zu behandelnden Körperregion abgegeben wird und wobei die Vorrichtung eine Spülvorrichtung zum Entfernen des Farbstoffüberschusses, also des Photosensitizers und eine Einsaugvorrichtung aufweist, die das Spülmittel und den darin gelösten Farbstoffüberschuß größtenteils wieder einsaugt und das Mittel zur Abgabe des Photosensitisers, vorzugsweise ein Schlauch, den lichtabgebenden Teil des Lichtmittels umhüllt, wobei das umhüllende Material zumindest teilweise das von dem Lichtmittel abgegebene Licht durchlässt.

2. Vorrichtung nach einem der vorhergehenden Ansprüche, **dadurch gekennzeichnet, dass** das Mittel zur Abgabe des Photosensitizers aus mindestens einem Schlauch besteht, welcher den Photosensitizer von einer Quelle über den Schlauch hin zu den zu behandelnden Körperregionen leitet, wobei der Schlauch mindestens einen Auslaß zur Abgabe des Photosensitizers, aber in seiner Maximalanzahl unbegrenzt viele Ausläße zur Abgabe des Photosensitizers aufweisen kann.

3. Vorrichtung nach einem der Ansprüche 1 bis 2, **dadurch gekennzeichnet, dass** das Mittel zur Abgabe des Photosensitizers, vorzugsweise ein Schlauch, den lichtabgebenden Teil des Lichtmittels umhüllt, wobei das umhüllende Material zumindest teilweise das von dem Lichtmittel abgegebene Licht durchlässt.

4. Vorrichtung nach einem der vorhergehenden Ansprüche, **dadurch gekennzeichnet, dass** das Lichtmittel mit dem Photosensitizer beschichtet wird.

5. Vorrichtung nach einem der vorhergehenden Ansprüche, **dadurch gekennzeichnet, dass** das Lichtmittel mit einem Material umhüllt wird, wobei das umhüllende Material aus einem Gewebe besteht, welches den Photosensitizer aufgenommen hat und diesen dann wieder an die zu behandelnden Körperregionen abgibt.

6. Vorrichtung zur Durchführung einer antibakteriellen photodynamischen Therapie nach einem der vorhergehenden Ansprüche, **dadurch gekennzeichnet, dass** die Vorrichtung der jeweiligen Form des zu behandelnden Körperraumes angepasst ist.

7. Vorrichtung zur Durchführung einer antibakteriellen photodynamischen Therapie nach einem der vorhergehenden Ansprüche, **dadurch gekennzeichnet, dass** die Spülvorrichtung einen Ultraschall-Vernebler aufweist.

8. Vorrichtung zur Durchführung einer antibakteriellen photodynamischen Therapie im Nasenraum nach einem der vorhergehenden Ansprüche, **dadurch gekennzeichnet, dass** die Vorrichtung y-förmig ausgestaltet ist und jedes der beiden oberen Enden der y-förmigen Vorrichtung eine eigene Spül- bzw. Einsaugvorrichtung und eine Lichtquelle aufweist.

9. Vorrichtung nach Anspruch 8, **dadurch gekennzeichnet, dass** die Spül- und Einsaugvorrichtung des einen Endes der Vorrichtung (1) dann spült, wenn die Spül- und Einsaugvorrichtung des anderen Endes der Vorrichtung (1) das Spülmittel einsaugt.

10. Vorrichtung nach einem der Ansprüche 8 und 9, **dadurch gekennzeichnet, dass** die Spülvorrichtung als Inhalationsvorrichtung ausgestaltet ist..

11. Vorrichtung zur Durchführung einer antibakteriellen photodynamischen Therapie im Mund- und Rachenraum nach einem der vorhergehenden Ansprüche, **dadurch gekennzeichnet, dass** die Vorrichtung die gleiche Form wie die Zahnleiste aufweist

12. Vorrichtung nach Anspruch 8, **dadurch gekennzeichnet, dass** die Spül- und Einsaugvorrichtung des einen Vorrichtungsendes dann spült, wenn die Spülund Einsaugvorrichtung des anderen Vorrichtungsendes das Spülmittel einsaugt.

13. Vorrichtung nach einem der Ansprüche 1 bis 7, **dadurch gekennzeichnet, dass** sie zylinderförmig ausgestaltet ist.

## Claims

1. An apparatus for carrying out antibacterial photodynamic therapy, **characterized in that** the apparatus comprises an illumination means and a means for dispensing the photosensitizer, and wherein
• the illumination means is an optical fiber emitting the light at the desired frequency through its side walls, and wherein the apparatus
• the means for dispensing the photosensitizer is adapted such that the photosensitizer is dispensed in spatial proximity to the body region to be treated, and wherein the apparatus comprises one flushing device for removing the excess dye, i.e. the photosensitizer, and comprises a suction device, which sucks most of the flushing fluid and the excess dye dissolved therein back in, and the means for dispensing the photosensitizer, preferably a tube, sheaths the light emitting portion of the illumination means, wherein the sheathing material at least partially transmits the light emitted by the illumination means.

2. The apparatus according to any one of the preceding claims, **characterized in that** the means for dispensing the photosensitizer comprises at least one tube, which conducts the photosensitizer from a source via the tube to the body regions to be treated, wherein the tube can comprise at least one outlet for dispensing the photosensitizer, but can also comprise an indefinite maximum number of outlets for dispensing the photosensitizer.

3. The apparatus according to any one of claims 1 to 2, **characterized in that** the means for dispensing the photosensitizer, preferably a tube, sheaths the light emitting portion of the illumination means, wherein the sheathing material at least partially transmits the light emitted by the illumination means.

4. The apparatus according to any one of the preceding claims, **characterized in that** the illumination means is coated with the photosensitizer.

5. The apparatus according to any one of the preceding claims, **characterized in that** the illumination means is sheathed with a material, wherein the sheathing material consists of a fabric, which has absorbed the photosensitizer and, in turn, dispenses the latter to the body regions to be treated.

6. An apparatus for carrying out antibacterial photodynamic therapy according to any one of the preceding claims, **characterized in that** the apparatus is adapted to the shape of each body cavity to be treated.

7. An apparatus for carrying out antibacterial photodynamic therapy according to any one of the preceding claims, **characterized in that** the flushing device comprises an ultrasonic nebulizer.

8. An apparatus for carrying out antibacterial photodynamic therapy in the nasal cavity according to any one of the preceding claims, **characterized in that** the apparatus has a y-shaped configuration and each of the two upper ends of the y-shaped apparatus has its own flushing and/or suction device and a light source.

9. The apparatus according to claim 8, **characterized in that** the flushing and/or suction device of one end of the apparatus (1) flushes whenever the flushing and/or suction device of the other end of the apparatus (1) sucks in the flushing fluid.

10. The apparatus according to any one of claims 8 and 9, **characterized in that** the flushing device is configured as an inhalation apparatus.

11. The apparatus for carrying out antibacterial photodynamic therapy in the oral and pharyngeal cavities according to any one of the preceding claims, **characterized in that** the apparatus (applicator) has the same form as the dental ridge.

12. The apparatus according to claim 8, **characterized in that** the flushing and/or suction device of one end of the apparatus flushes whenever the flushing and/or suction device of the other end of the apparatus sucks in the flushing fluid.

13. The apparatus according to any one of claims 1 to 7, **characterized in that** it has a cylindrical configuration.

## Revendications

1. Dispositif pour la mise en place d'une thérapie photo-dynamique antibactérienne, dans lequel le dispositif comportant un moyen lumineux et un moyen pour le dépôt de l'agent photo-sensibilisant, et dans lequel
• le moyen lumineux est une fibre optique émettant la lumière dans la fréquence souhaitée par ses parois latérales, et
• le moyen pour le dépôt de l'agent photo-sensibilisant est conçu de manière à ce que l'agent photo-sensibilisant soit déposé à proximité de la région corporelle à traiter, et dans lequel le dispositif comporte un dispositif de rinçage pour l'élimination de l'excédent de colorant, donc de l'agent photo-sensibilisant, ainsi qu'un dispositif d'aspiration aspirant de nouveau la majeure partie du liquide de rinçage et de l'excédent de colorant dissolu dans celui-ci, et le moyen pour le dépôt de l'agent photo-sensibilisant, de préférence un tuyau, enveloppe la partie émettrice de lumière du moyen lumineux, le matériau enveloppant laissant au moins partiellement passer la lumière émise par le moyen lumineux.

2. Dispositif selon l'une des revendications précédentes, **caractérisé en ce que** le moyen pour le dépôt de l'agent photo-sensibilisant est constitué d'au moins un tuyau, lequel guide l'agent photo-sensibilisant à partir d'une source jusqu'aux régions corporelles à traiter, par le biais du tuyau, le tuyau pouvant comporter au moins une sortie pour le dépôt de l'agent photo-sensibilisant, mais pouvant comporter un nombre illimité de sorties pour le dépôt de l'agent photo-sensibilisant, quant au nombre maximal.

3. Dispositif selon l'une des revendications 1 à 2, **caractérisé en ce que** le moyen pour le dépôt de l'agent photo-sensibilisant, de préférence un tuyau, enveloppe la partie émettrice de lumière du moyen lumineux, le matériau enveloppant laissant au moins partiellement passer la lumière émise par le moyen lumineux.

4. Dispositif selon l'une des revendications précédentes, **caractérisé en ce que** le moyen lumineux est revêtu avec l'agent photo-sensibilisant.

5. Dispositif selon l'une des revendications précédentes, **caractérisé en ce que** le moyen lumineux est enveloppé dans un matériau, le matériau enveloppant étant constitué d'un tissu capable d'absorber l'agent photo-sensibilisant, puis de déposer celui-ci sur les régions corporelles à traiter.

6. Dispositif pour la mise en place d'une thérapie photo-dynamique antibactérienne selon l'une des revendications précédentes, **caractérisé en ce que** le dispositif est adapté à la forme respective de l'espace corporel à traiter.

7. Dispositif pour la mise en place d'une thérapie photo-dynamique antibactérienne selon l'une des revendications précédentes, **caractérisé en ce que** le dispositif de rinçage comporte un nébuliseur à ultrasons.

8. Dispositif pour la mise en place d'une thérapie photo-dynamique antibactérienne selon l'une des revendications précédentes, **caractérisé en ce que** le dispositif est conçu en forme de y, et chacune des deux extrémités supérieures du dispositif en forme de y comporte un dispositif de rinçage et d'aspiration propre et une source de lumière.

9. Dispositif selon la revendication 8, **caractérisé en ce que** le dispositif de rinçage et d'aspiration de l'une des extrémités du dispositif (1) assure un rinçage lorsque le dispositif de rinçage et d'aspiration de l'autre extrémité du dispositif (1) aspire le liquide de rinçage.

10. Dispositif selon l'une des revendications 8 et 9, **caractérisé en ce que** le dispositif de rinçage est conçu comme un dispositif d'inhalation.

11. Dispositif pour la mise en place d'une thérapie photo-dynamique antibactérienne dans la cavité buccale et pharyngienne selon l'une des revendications précédentes, **caractérisé en ce que** le dispositif présente la même forme que la lame dentaire.

12. Dispositif selon la revendication 8, **caractérisé en ce que** le dispositif de rinçage et d'aspiration de l'une des extrémités du dispositif assure un rinçage lorsque le dispositif de rinçage et d'aspiration de l'autre extrémité du dispositif aspire le liquide de rinçage.

13. Dispositif selon l'une des revendications 1 à 7, **caractérisé en ce qu'**il est conçu en forme de cylindre.
